(19) Europäisches Patentamt
European Patent Office
Office européen des brevets

(11) Publication number: 0 014 493 B1

# (12) EUROPEAN PATENT SPECIFICATION

(45) Date of publication of patent specification: 29.12.82

(21) Application number: 80200039.8

(22) Date of filing: 16.01.80

(51) Int. Cl.³: C 07 C 69/712, C 07 C 59/68, C 07 D 213/30, C 07 C 79/46 //C07C67/31, A61K31/215

## (54) Chlorobenzyl phenoxy alkanoic compounds.

(30) Priority: 25.01.79 US 6542

(43) Date of publication of application: 20.08.80 Bulletin 80/17

(45) Publication of the grant of the patent: 29.12.82 Bulletin 82/52

(84) Designated Contracting States: AT BE CH DE FR GB IT LU NL SE

(56) References cited:
FR - A - 2 255 891
FR - A - 2 267 302

(73) Proprietor: SIEGFRIED AKTIENGESELLSCHAFT
CH-4800 Zofingen (CH)

(72) Inventor: Thiele, Kurt, Dr.
Rebbergstrasse 47
CH-4800 Zofingen (CH)
Inventor: Ahmed, Quazi, Dr.
Rigiweg 5
CH-4800 Zofingen (CH)
Inventor: Adrian, Rudolf, Dr.
Rümlisberg 470
CH-4803 Vordemwald (CH)
Inventor: Jahn, Ulrich, Dr.
Kirchmoosstrasse 13
CH-4800 Zofingen (CH)

(74) Representative: Ritscher, Thomas, Dr. et al,
RITSCHER & SEIFERT Auf der Mauer 4
CH-8001 Zürich (CH)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 014 493 B1

Chlorobenzyl phenoxv alkanoic compounds

The invention relates to novel diphenyl methane derivatives having improved pharmacological properties when used for the therapy of hypercholesterolemia and hyperlipidemia and more particularly to chlorobenzyl phenoxy alkanoic compounds wherein the oxyalkanoic moiety is in ortho position of the phenyl ring and may have an asymmetrical carbon atom as the link between the phenoxy moiety and the carboxylic function.

Ethyl p-chlorophenoxyisobutyrate (generic name: Clofibrate) having the formula (20)

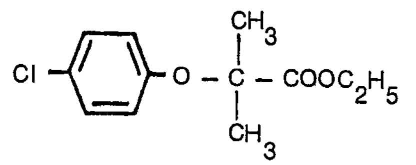

(20)

disclosed by Glynn M. Jones et al in U.S. Patent 3,262,850 is a widely accepted therapeutic agent used for reducing the concentration of cholesterol in the blood serum. The relatively high toxicity ($LD_{50}$ 2350 mg/kg, mice) of Clofibrate and the necessity to use it in relatively high doses because of its low therapeutic effectiveness has triggered the search for other therapeutic agents suitable for treatment of hypercholesterolemia and having improved pharmacological properties.

Fukami et al in German Published Specification 2,356,655 discloses anti-hypercholerolemic compounds of the formula (30)

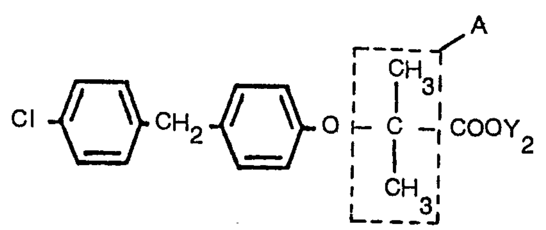

(30)

in which $Y^2$ is lower alkylk or a N,N-diloweralkyl substituted aminoalkylene gorup.

Applicants have previously disclosed (e.g. in German Published Specification 2,461,069) that certain asymmetrical homologues of the Fukami compounds (30) of the formula (40)

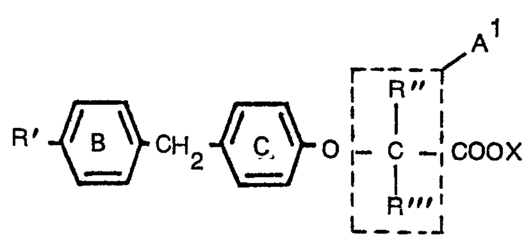

(40)

in which R′ is hydrogen, halogen, lower alkyl or lower alkoxy and X is lower alkyl, provide for substantial improvements, notably an improved therapeutic ratio ($LD_{50}$ divided by cholesterol lowering effectiveness) if link $A^1$ is asymmetrical; preferably R″ and R‴ are different lower alkyls having a combined total of at least 3 carbon atoms, e.g. methyl/ethyl, methyl/propyl, etc.

When reviewing other parameters except link $A^1$ of the above formula (40) compounds, both the art referred to above as well as experimental evidence reported below seemed to indicate that the linear molecular form of the formula (40) structure between link $A^1$ and rina B was critical, notably with regard to the para-position of the methylene and the oxy substituent of ring C. For example, while some non-linear structures are disclosed in the Jones Patent mentioned above as well as in U.S. Patent 3,362,997 to Bolhofer, and while non-linear structures of the general class of compounds referred to above are disclosed as herbicides in U.S. Patent 4,088,474 to Matterstock et al, it is but the linear structure that has been used for cholesterol-lowering compounds according to the pertinent art.

On the other hand, experimental evidence indicates that relatively low toxicity data (i.e. high $LD_{50}$ values) of compounds of the above classes found to have satisfactory cholesterol-lowering effectiveness (i.e. low $ED_{25}$ values) may not be sufficient criterion in the evaluation of an optimum balance of properties. One generally accepted criterion in the context of drug-testing is the effect of the

drug on size and weight of the liver, notably the hepatomegalitic or liver weight increasing effect. This factor is typically measured in terms of the increase of the weight of the hepata (liver) of test animals (e.g. rats) after a period of drug administration. It has been observed that compounds believed to be suitable for treatment of hypercholesterolemia or hyperlipidemia may have an undesirable high hepatomegalitic effect, i.e. cause a significant or even substantial increase of the liver weight of the test animals after a period of drug administration even though the $LD_{50}$ toxicity of such compounds may be acceptably low.

Accordingly, it is the aim of this invention to provide for novel compounds suitable for the therapy of hypercholesterolemia and hyperlipidemia, said novel compounds having an improved balance of pharmacological properties, i.e. an acceptably low toxicity combined with a satisfactory effectiveness in reducing the concentration of cholesterol in the blood and no or very low effects upon the liver weight.

According to the present invention we have found a group of novel compounds of the formula (1)

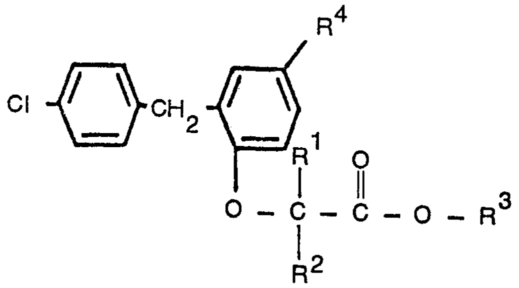

(1)

wherein $R^1$ and $R^2$ are the same or different lower alkyls, i.e. having from 1 to 4 carbon atoms each when $R^4$ is hydrogen; according to a preferred embodiment, one of $R^1$ and $R^2$ is methyl while the other is ethyl, propyl or butyl; the combination of methyl/ethyl or methyl/propyl for $R^1$, $R^2$ is particularly preferred. When $R^1$ and $R^2$ are different alkyls, the carbon atom between $R^1$ and $R^2$ is asymmetrical. Accordingly, the invention includes the D-isomers, the L-isomers and the D,L-racemates of formula (1) compounds when $R^1$ differs from $R^2$. When $R^4$ is nitro, $R^1$ and/or $R^2$ may also represent hydrogen.

Preferably, $R^3$ is alkyl of from 1 to 4 carbon atoms, ethyl being a particularly preferred example; $R^3$ can also represent hydrogen or the ion of a pharmaceutically acceptable metal, e.g. sodium, potassium, calcium, magnesium and aluminum; further and according to another preferred embodiment, $R^3$ can stand for alkyl ($C_1$—$C_4$ preferred), that is, substituted with a nitrogen-containing basic group, e.g. pyridyl methyl, i.e. a group of the formula

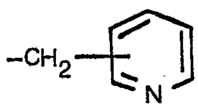

or an aminoalkylene (amino-lower alkylene being preferred) group

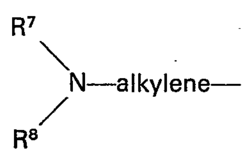

in which $R^7$ and $R^8$ are the same or different and are hydrogen and/or alkyl, preferably lower alkyl.

Compounds of formula (1) wherein $R^3$ includes a nitrogen-containing basic group may be in the form of salts of addition with adics. For pharmacological use such salts of addition will be those with pharmacologically acceptable acids including mineral or inorganic acids such as hydrocholric, nitric, sulphuric and phosphoric acid and the like, as well as organic acids such as lactic, citric and tartaric acid and the like. Many other pharmacologically acceptable acids are known in the art and can be used for acid addition salts of the inventive compounds wherein $R^3$ includes a nitrogen-containing basic group, such as when $R^3$ is pyridyl methyl. 3-pyridiyl methyl (also called nicotinyl) is a preferred specific example of such an $R^3$.

$R^4$ in formula (1) is a hydrogen atom or the nitro group. Hydrogen is preferred for $R^4$ in many cases.

The compounds of formula (2)

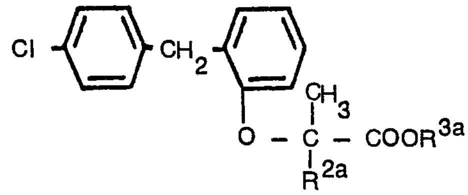

(2)

wherein $R^{2a}$ is ethyl or propyl, $R^{3a}$ is lower alkyl, preferably ethyl, or pyridyl methyl (including salts of addition) are preferred examples of a compound according to the invention.

Also preferred are the compounds of formula (3)

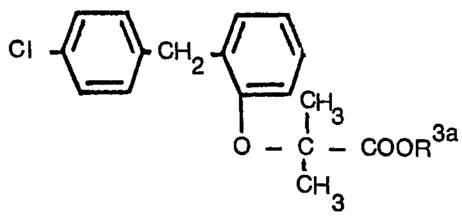

(3)

wherein $R^{3a}$ is as defined above for formula (2).

Further compounds of the invention are those of formula (1a)

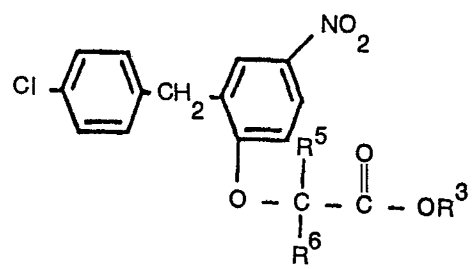

(1a)

wherein $R^3$ is hydrogen, a lower alkyl optionally substituted with a nitrogen-containing basic group or an ion of a pharmaceutically acceptable metal, $R^5$ and $R^6$ are the same or different and are hydrogen atoms or lower alkyls, and the acid addition salts of said formula (1a) compounds with pharmaceutically acceptable acids when $R^3$ comprises a nitrogen-containing basic group.

Again, the carbon atom between the oxy group and the carboxyl group may be, and preferably is, asymmetrical and, again, the invention includes the racemates as well as the pure stereoisomers.

A specific method of preparing the subject compounds will be explained below in the examples but other methods could be used to produce the compounds according to the invention.

According to a first general method, the subject compounds can be obtained by condensing a phenol of formula (4)

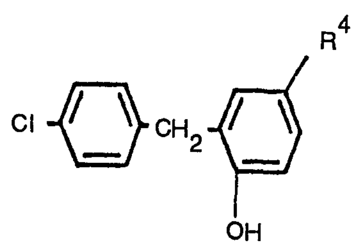

(4)

wherein $R^4$ is hydrogen or nitro or the corresponding phenolate such as an alkali metal phenolate, e.g. the sodium or potassium phenolate, with a compound of formula (5) or (5')

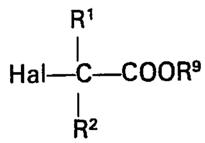 or 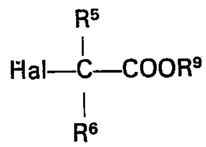

(5) (5′)

wherein Hal is a halogen atom such as chlorine, bromine, etc.; $R^1$, $R^2$, $R^5$ and $R^6$ are defined as above and $R^9$ is $R^3$ as defined above or hydrogen or a group that can be converted into $R^3$, e.g. by transesterification. Depending upon the significance of $R^9$, the product of condensing compounds (4) and (5) or (5′) is the target compound or will have to be converted into the target compound, e.g. by esterification with the corresponding alcohol $R^3OH$, when $R^9$ is hydrogen, or by transesterification or by forming the appropriate salt.

The use of a base suitable as an acid-binding agent in the condensation of compounds (4) and (5) or (5′) may be advantageous but is not believed to be critical. Preferably, condensation of (4) and (5) or (5′) is effected in an organic solvent such as xylene, generally under substantially anhydrous conditions and at elevated temperatures, e.g. up to reflux temperatures of the solvent.

The condensation product can be recovered from the reaction mixture by evaporating the solvent and destillation and/or recrystallization of the residue. The condensation product is generally obtained in the form of the D,L-racemate. The D- and L-stereoisomers could be obtained from the racemate, e.g. by chromatography, but such separation is not believed to be critical.

Specific examples of methods suitable for producing the novel compounds will be given below. These examples are illustrative and not to be understood as limiting the scope of the invention in any way. In the examples, percentages are by weight. Elemental analysis data are in percent by weight.

## Example I

*Preparation of ethyl D,L-2-methyl-2-[o-(p′-chlorobenzyl)-phenoxy]-butyrate* formula 2, $R^{2a}$ = ethyl, $R^{3a}$ = ethyl)

A solution of 4′-chloro-2-hydroxy-diphenyl-methane (218.6 g, 1.0 mol) in 1200 ml of dry xylene was added to freshly prepared sodium ethoxide (68.05 g, 1.0 mol) and the mixture was refluxed for 2 hours. A solution of ethyl D,L-2-bromo-2-methylbutyrate (209.10 g, 1.0 mol) in 50 ml of dry xylene was added carefully in a dropwise manner to the mixture during a period of 15 minutes. The reaction was continued for 22 hours and then allowed to cool to room temperature (20—25°C). The reaction mixture was washed twice with 300 ml of 1 n NaOH solution. The organic phase was washed free of alkali with water, dried (over anhydrous magnesium sulphate), and evaporated under reduced pressure to give 196.30 g of a light yellow oil. The product was dissolved in 100 ml of n-hexane. The solution obtained was filtered through a column of basic alumina (500 g) and the colorless oil was distilled to give 98.05 g of the target product, b.p. 156—157°C/0.01 mm Hg.

Analysis calculated for

| | | | |
|---|---|---|---|
| $C_2OH_{23}C1O_3$: | C 69.25, | H 6.68, | Cl 10.22 |
| found: | C 69.23, | H 6.82, | Cl 10.30. |

## Example II

*Preparation of ethyl D,L-2-methyl-2-[o-(p′-chlorobenzyl)-phenoxy]-valerate* (formula 2, $R^{2a} = C_3H_7$, $R^{3a} = C_2H_5$)

A solution of 4′-chloro-2-hydroxy-diphenyl-methane (109.30 g, 0.5 mol) in 600 ml of dry xylene was added to sodium ethoxide (34.0 g, 0.50 mol) and the mixture was refluxed for 2 hours. A solution of ethyl D,L-2-bromo-2-methyl-valerate (111.60 g, 0.50 mol) in 25 ml of dry xylene was added dropwise to the mixture during a period of 5 minutes. The reaction was continued for 22 hours and was worked-up as in Example I to yield 95.0 g of an oil product. Distillation of the product gave 70.0 g of the target product in the form of a colorless oil, b.p. 166—168°C/0.01 mm Hg.

Analysis calculated for

| | | | |
|---|---|---|---|
| $C_{21}H_{25}ClO_3$: | C 69.89, | H 6.98, | Cl 9.82 |
| found: | C 70.00, | H 6.91, | Cl 9.66. |

Example III

*Preparation of 3-pyridyl methyl ester of D,L-2-methyl-2-[o-(p'-chlorobenzyl)-phenoxy]-butyric acid (hydrochloride)* (formula 2, $R^{2a} = C_2H_5$, $R^{3a} =$

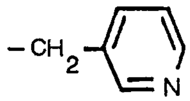 • HCl)

To a solution of D,L-2-methyl-2-[o-(p'-chlorobenzyl)-phenoxy]-butyryl-chloride (26.64 g, 0.079 mol) in 50 ml of dry benzene and 7.7 ml of dry pyridine there was added, in a dropwise manner, a solution of 3-hydroxymethyl-pyridine (8.62 g, 0.079 mol) in 10 ml of dry benzene. The mixture was stirred at room temperature for 20 hours. Removal of the solvents under vacuum yielded a brown residue which was treated with 120 ml of 10% $KHCO_3$ solution. The alkaline mixture was extracted with dichloromethane, washed with water and dried over anhydrous magnesium sulphate. The residue obtained after removal of the solvent was dissolved in cyclohexane and filtered through a column of 50.0 g of basic alumina. The light yellow colorless residue was then dissolved in ether and treated with HCl/ether. The hydrochloride was recrystallized twice from acetone to give 15.0 g of target product in the form of shiny needles, mp. 114—115°C.

Analysis calculated for:

| | | | | |
|---|---|---|---|---|
| $C_{24}H_{24}ClNO_3HCl$: | C 64.58, | H 5.65, | N 3.14, | Cl 15.89 |
| found: | C 64.47, | H 5.53, | N 3.38 | Cl 15.89. |

Example IV

*Preparation of ethyl 2-methyl-2-[o-(p'-chlorobenzyl)-phenoxy]-propionate* (formula 3, $R^{3a} = C_2H_5$)

A solution of 4'-chloro-2-hydroxy-diphenyl-methane (109.30 g, 0.50 mol) in 600 ml of dry xylene was added to sodium ethoxide (34.0 g, 0.50 mol) and the mixture was heated under reflux for 2 hours. Thereafter, a solution of ethyl 2-bromo-isobutyrate (97.50 g, 0.5 mol) in 25 ml of dry xylene was added dropwise to the mixture during a period of 10 minutes. After 22 hours, the reaction mixture was worked-up as in Example I to yield an oily product. Distillation yielded 70.0 g of the target product in the form of a colorless oil, b.p. 156°C/0.01 mm Hg.

Analysis calculated for:

| | | | |
|---|---|---|---|
| $C_{19}H_{21}C1O_3$: | C 68.56, | H 6.36, | Cl 10.65 |
| found: | C 68.26, | H 6.45, | Cl 10.45. |

Example V

*Preparation of 3-pyridyl methyl ester of 2-methyl-2-[o-(p'-chlorobenzyl)-phenoxy]-propionic acid (hydrochloride)* (formula 3, $R^3 =$

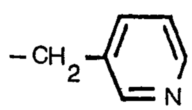 • HCl)

A solution of 3-hydroxymethyl-pyridine (8.07 g, 0.074 mol) in 10 ml of dry benzene was added to a solution of 2-methyl-2-[o-(p'-chlorobenzyl)-phenoxy]-propionyl chloride (23.96 g, 0.074 mol) in 50 ml of dry benzene and 7 ml of dry pyridine. The mixture was stirred at room temperature for 20 hours. Work-up was as in Example I to yield a crystalline residue that was recrystallized from acetone to yield 12 g of the target product in the form of shiny needles, m.p. 119—121°C.

Analysis calculated for

| | | | | |
|---|---|---|---|---|
| $C_{23}H_{22}ClNO_3HCl$: | C 63.90, | H 5.36 | N 3.24 | Cl 16.41 |
| found: | C 63.82, | H 5.30, | N 3.64, | Cl 16.47. |

Examples VI to X

The following compounds were prepared according to the above methods:

VI: The compound of formula (6)

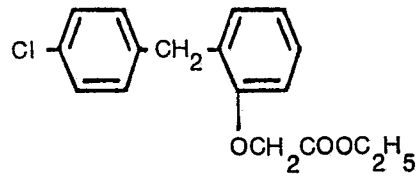

(6)

VII: The compound of formula (7)

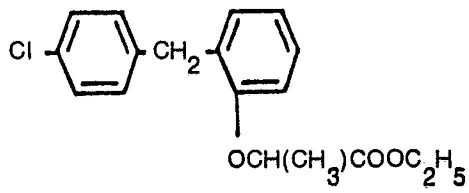

(7)

VIII: The compound of formula (8)

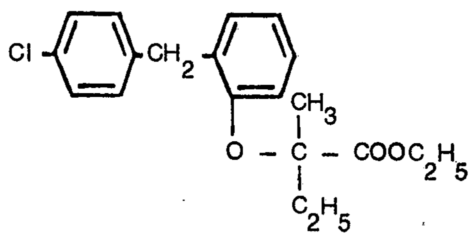

(8)

IX: The compound of formula (9)

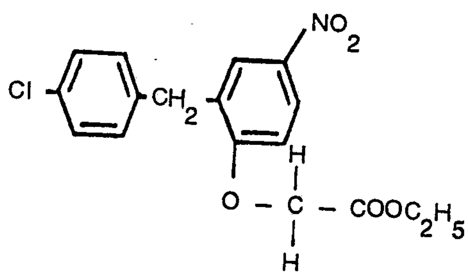

(9)

X: The compound of formula (10)

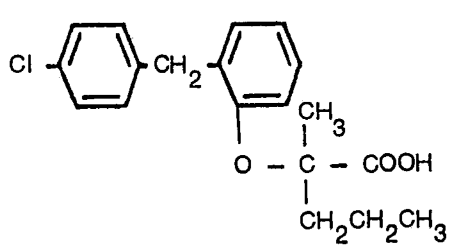

(10)

Pharmacological Data

The compounds of Examples I to X were tested to evaluate their pharmacological effectiveness. Specifically, their toxicity ($LD_{50}$), cholesterol-lowering effectiveness ($E_1D_{25}$) and their liver weight

increasing activity ($E_2D_{25}$) were tested on animals according to standard methods. The methods are reviewed below and the results are reported in Table I below.

(A) Acute toxicity as $LD_{50}$ lethal dose is given in milligrams of the tested substance per kilogram of body weight (mg/kg) of the test animals; the data reported were determined by the conventional method according to Litchfield and Wilcoxon (J. Pharmacol. *95*/1949/p, 99ff) on mice; the test substance was administered orally once and the mice were observed for a period of 7 days following oral administration or longer if required. At least three different doses were tested for each $LD_{50}$ value given and 10 animals were used for testing of each dose; five test animals were kept in one standard "Macrolon" cage. The test substance was administered as a suspension in aqueous (3—5% by weight) Gum Arabic via a probe into the stomach quantities of 10 ml of the suspension per kilogram body weight. As in conventional, $LD_{50}$ is that dose of the test substance in mg/kg body weight which causes death of 50% of the test animals within the observation period. Higher $LD_{50}$ values indicate lower toxicity and vice versa.

(B) The effective dose $E_1D_{25}$ is the amount of test substance in milligram per kilogram body weight of the test animals (male Wistar rats, 100—200 g body weight, eight to ten animals per dosis) that causes a 25% by weight reduction of the average cholesterol level in the blood serum of test animals that had received the test substance, compared with the cholesterol level in the blood serum of the control group. The test substances were administered orally via a probe in the manner set forth above for the $LD_{50}$ toxicity, i.e. as suspension in aqueous Gum Arabic. Each test animal was administered daily with 10 ml of the suspension per kilogram body weight. The animals of the control group were administered daily with each 10 ml of the aqueous Gum Arabic per kilogram of body weight without the test substance. The daily dose was administered for 5 subsequent days, resumed after a two-day weekend interval and continued for 5 additional days. After a total test period of two weeks, both the test and the control animals were sacrificed and the blood serum was recovered from the bodies for analysis.

Cholesterol analysis of the serum recovered was carried out according to the standard method of M. Richterich (cf. Monography by M. Richterich, "Klinische Chemie", Verlag Karger, Basel/New York, 1965, p. 232) and the results were evaluated by means of a cholesterol level/dose diagram. The $E_1D_{25}$ values were taken from the diagram thus obtained. Lower $E_1D_{25}$ values indicate higher activity, and vice versa.

(C) The effective dose $E_2D_{25}$ is the amount of test substance in milligram per kilogram body weight of the test animals (male Wistar rats, 100—200 g body weight, eight to ten animals per dose) that causes a 25% increase of the liver weight/body weight ratio (relative liver weight) of test animals that had received the test substance, compared with the liver weight/body weight ratio of the control group. Administration was effected substantially in the manner described above for $E_1D_{25}$. The relative liver weight was determined from the bodies of the sacrificed animals and the control animals. The actual increase of the relative liver weight was evaluated graphically and the $E_2D_{25}$ was obtained by interpolation from the graph.

TABLE I

| Compound | $LD_{50}$ | $E_1D_{25}$ | $E_2D_{25}$ |
|---|---|---|---|
| Example I | >10,000 | 13 | ≫300 |
| Example II | >10,000 | ca. 100 | ≫100 |
| Example III | >3000 | 12 | ≫100 |
| Example IV | >10,000 | ca. 50 | ≫100 |
| Example V | >3000 | 4 | ≫100 |
| Example VI | ca. 3000 | >300 | ≫300 |
| Example VII | ≫3000 | >100 | ≫100 |
| Example VIII | not measured | 40 | 16 |
| Example IX | >3000 | 26 | ≫100 |
| Example X | >1000 | ca. 30 | ≫100 |
| Substance A[1] | 8000 | 12.1 | 8.8 |
| Substance B[2] | 2350 | 151 | 136 |

(1) Substance A is the ethyl ester of 2-methyl-2-[4′-(4′-chlorobenzyl)-phenoxy]-butyric acid prepared according to Example 11 of German Published Patent Application 2,461,069, cf. formula (40) above.

(2) Substance B is the ethyl ester of p-chlorophenoxyisobutyric acid (Clofibrate) disclosed in U.S. Patent 3,262,850, cf. formula (20) above.

From the results reported in Table I it is apparent that the compounds of Examples I, III, IV, V, IX and X are preferred, the compounds of Examples I, III and V being particularly preferred because of low toxicity combined with high cholesterol-lowering effectiveness and very low liver enlargement activities.

On the other hand, the comparative compounds of Examples VI and VII have a substantially reduced cholesterol-lowering effect while the compound of Example VIII (the metaisomer of Example I) has an undesirably high liver enlargement activity.

Substances A and B are prior art compounds shown for comparative purposes only.

Inventive compounds of the above formulae (1), (1a), (2) and (3) are suitable for therapeutic purposes and can be used in a manner analoguous to that disclosed in U.S. Patent 3,262,850.

Hence, the compounds according to this invention have commercial utility in pharmaceutical compositions for treatment of hypercholesterolemia and hyperlipidemia of the type comprising at least one inventive compound and, generally, a pharmacologically acceptable carrier of the type known per se, e.g. as disclosed in U.S. Patent 3,262,850.

**Claims for the Contracting States: BE CH DE FR GB IT LU NL SE**

1. Compounds of the formula (1)

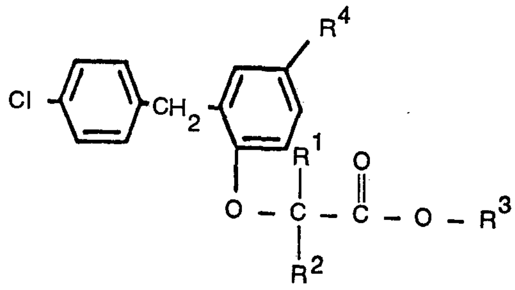

(1)

wherein $R^1$ and $R^2$ are the same or different lower alkyls when $R^4$ is hydrogen, or wherein $R^1$ and $R^2$ are the same or different and are hydrogen or lower alkyls when $R^4$ is nitro; $R^3$ is hydrogen, a lower alkyl optionally substituted with a nitrogen-containing basic group, or an ion of a pharmaceutically acceptable metal; and the acid addition salts of said formula (1) compounds with pharmaceutically acceptable acids when $R^3$ comprises a nitrogen-containing basic group.

2. The compounds of claim 1 wherein $R^3$ is ethyl, propyl or butyl, or is pyridyl methyl.

3. The compounds of claims 1 or 2 wherein $R^1$ is methyl, ethyl or propyl.

4. The compounds of any of claims 1, 2 or 3 wherein $R^2$ is methyl, ethyl or propyl.

5. The compounds of claim 1 wherein $R^1$ is methyl, $R^2$ is ethyl or propyl, $R^3$ is ethyl or pyridyl methyl and $R^4$ is hydrogen.

6. The compounds of any of claims 2—4 wherein $R^1$ and $R^2$ are different lower alkyls, said compounds being in the form of the D,L-racemate or in the form of the D- or L-stereo-isomers.

7. The compounds of claims 1 wherein $R^1$ and $R^2$ are methyl, $R^3$ is lower alkyl or pyridyl methyl and $R^4$ is hydrogen.

8. The compounds of claim 7 wherein $R^3$ is ethyl.

**Claims for the Contracting State: AT**

1. A process for producing compounds of the formula (1)

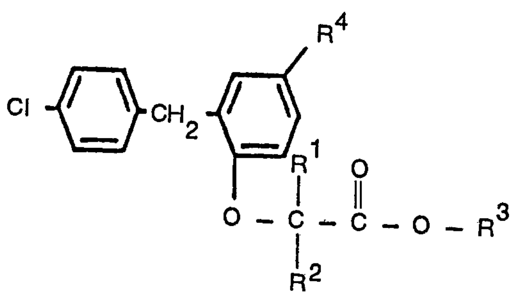

(1)

wherein $R^1$ and $R^2$ are the same or different lower alkyls when $R^4$ is hydrogen, or wherein $R^1$ and $R^2$ are the same or different and are hydrogen or lower alkyls when $R^4$ is nitro; $R^3$ is hydrogen, a lower alkyl optionally substituted with a nitrogen-containing basic group, or an ion of a pharmaceutically acceptable metal; and the acid addition salts of said formula (1) compounds with pharmaceutically acceptable acids when $R^3$ comprises a nitrogen-containing basic group, characterized by condensing a phenol of formula (4)

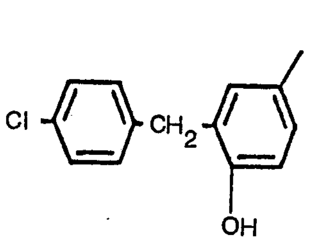

(4)

wherein $R^4$ is as defined above or a phenolate of said formula (4) phenol with a compound of formula (5)

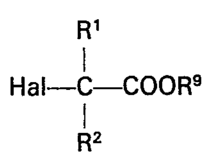

(5)

wherein Hal is a halogen atom, $R^1$ and $R^2$ are as defined above and $R^9$ is $R^3$ as defined above or is a group that can be converted into $R^3$ and in the latter case subsequently converting the product into the target compound of formula (1).

2. The process of claim 1, characterized by forming the acid addition salts of said formula (1) compounds with pharmaceutically acceptable acids when $R^3$ comprises a nitrogen-containing basic group.

3. The process of claim 1 wherein $R^3$ is ethyl, propyl or butyl, or is pyridyl methyl.

4. The process of claim 1, 2 or 3 wherein $R^1$ is methyl, ethyl or propyl.

5. The process of any of claims 1, 2, 3 or 4 wherein $R^2$ is methyl, ethyl or propyl.

6. The process of claim 1 or 2 wherein $R^1$ is methyl, $R^2$ is ethyl or propyl, $R^3$ is ethyl or pyridyl methyl and $R^4$ is hydrogen.

7. The process of any of claims 1—5 wherein $R^1$ and $R^2$ are different lower alkyls, said compounds being in the form of the D,L-racemate or in the form of the D- or L-stereo-isomers.

8. The process of claim 1 or 2 wherein $R^1$ and $R^2$ are methyl, $R^3$ is lower alkyl or pyridyl methyl and $R^4$ is hydrogen.

9. The process of claim 8 wherein $R^3$ is ethyl.

**Revendications pour les Etats Contractants: BE CH DE FR GB IT LU NL SE**

1. Composés de formule (1)

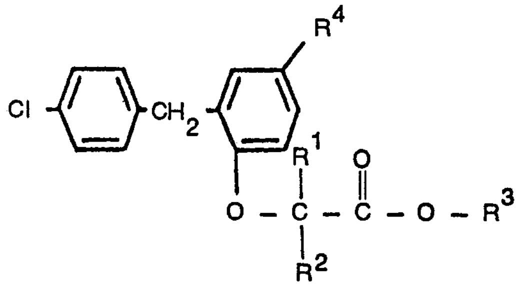

(1)

dans laquelle $R^1$ et $R^2$ sont des groupes alkyles inférieurs identiques ou différents lorsque $R^4$ est un atome d'hydrogène, ou dans laquelle $R^1$ et $R^2$ sont identiques ou différents et représentent un atome d'hydrogène ou des groupes alkyles inférieurs lorsque $R^4$ est un groupe nitro, $R^3$ est un atome d'hydrogène, un groupe alkyle inférieur éventuellement substitué par un groupe basique contenant de l'azote, ou un ion d'un métal pharmaceutiquement acceptable; et les sels d'addition des composés de formule (1) avec des acides pharmaceutiquement acceptables lorsque $R^3$ est un groupe basique contenant de l'azote.

2. Composés selon la revendication 1, caractérisés en ce que $R^3$ est un groupe éthyle, propyle ou butyle, ou est le groupe pyridylméthyle.

3. Composés selon l'une des revendications 1 et 2, caractérisés en ce que $R^1$ est un groupe méthyle, éthyle ou propyle.

4. Composés selon l'une quelconque des revendications 1 à 3, caractérisés en ce que $R^2$ est un groupe méthyle, éthyle ou propyle.

5. Composés selon la revendication 1, caractérisés en ce que $R^1$ est un groupe méthyle, $R^2$ est un groupe éthyle ou propyle, $R^3$ est un groupe éthyle ou pyridylméthyle, et $R^4$ est un atome d'hydrogène.

6. Composés selon l'une quelconque des revendications 2 à 4, caractérisés en ce que $R^1$ et $R^2$ sont des groupes alkyles inférieurs différents, les composés étant sous forme du racémate D,L ou sous forme des stéréoisomères D ou L.

7. Composés selon la revendication 1, caractérisés en ce que $R^1$ et $R^2$ sont des groupes méthyle, $R^3$ est un groupe alkyle inférieur ou pyridylméthyle et $R^4$ est un atome d'hydrogène.

8. Composés selon la revendication 7, caractérisés en ce que $R^3$ est un groupe éthyle.

**Revendications pour l'Etat Contractant: AT**

1. Procédé pour la préparation de composés de formule (1)

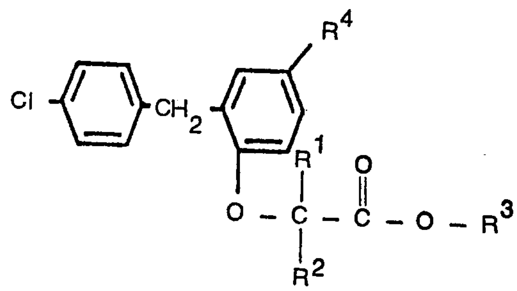

(1)

dans laquelle $R^1$ et $R^2$ sont des groupes alkyles inférieurs identiques ou différents lorsque $R^4$ est un atome d'hydrogène, ou dans laquelle $R^1$ et $R^2$ sont identiques ou différents et représentent un atome d'hydrogène ou des groupes alkyles inférieurs lorsque $R^4$ est un groupe nitro, $R^3$ est un atome d'hydrogène, un groupe alkyle inférieur éventuellement substitué par un groupe basique contenant de l'azote, ou un ion d'un métal pharmaceutiquement acceptable; et des sels d'addition des composés de formule (1) avec des acides pharmaceutiquement acceptables lorsque $R^3$ est un groupe basique contenant de l'azote, caractérisé par la condensation d'un phénol de formule (4)

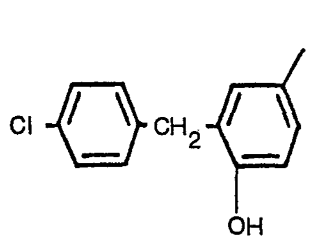

(4)

dans laquelle $R^4$ est tel que défini ci-dessus, ou d'un phénolate d'un phénol de formule (4) ci-dessus avec un composé de formule (5)

$$\text{Hal}-\underset{R^2}{\overset{R^1}{\underset{|}{\overset{|}{C}}}}-COOR^9 \qquad (5)$$

dans laquelle Hal représente un atome d'halogène, $R^1$ et $R^2$ sont tels définis précédemment et $R^9$ est $R^3$ tel que défini précédemment ou un groupe qui peut être transformé en $R^3$, et que l'on transforme ensuite, dans le dernier cas, le produit en le composé voulu de formule (1).

2. Procédé selon la revendication 1, caractérisé en ce que l'on produit les sels d'addition des composés de formule (1) avec des acides pharmaceutiquement acceptables lorsque $R^3$ est un groupe basique contenant de l'azote.

3. Procédé selon la revendication 1, caractérisé en ce que $R^3$ est un groupe éthyle, propyle ou butyle, ou est le groupe pyridylméthyle.

4. Procédé selon l'une des revendications 1, 2 ou 3, caractérisé en ce que $R^1$ est un groupe méthyle, éthyle ou propyle.

5. Procédé selon l'une ou plusieurs des revendications 1, 2, 3 ou 4, caractérisé en ce que $R^2$ est groupe méthyle, éthyle ou propyle.

6. Procédé selon la revendication 1 ou 2, caractérisé en ce que $R^1$ est un groupe méthyle, $R^2$ est un groupe éthyle ou propyle, $R^3$ est un groupe éthyle ou pyridylméthyle, et $R^4$ est un atome d'hydrogène.

7. Procédé selon l'une ou plusieurs des revendications 1 à 5, caractérisé en ce que $R^1$ et $R^2$ sont des groupes alkyles inférieurs différents, les composés étant sous forme du racémate D,L ou sous forme des stéréoisomères D ou L.

8. Procédé selon la revendication 1 ou 2, caractérisé en ce que $R^1$ et $R^2$ sont des groupes méthyle, $R^3$ est un groupe alkyle inférieur ou pyridylméthyle et $R^4$ est un atome d'hydrogène.

9. Procédé selon la revendication 8, caractérisé en ce que $R^3$ est un groupe éthyle.

**Patentansprüche für die Vertragsstaaten: BE CH DE FR GB IT LU NL SE**

1. Verbindungen der Formel (1)

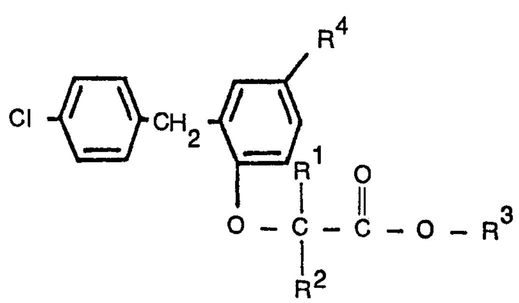

(1)

in welcher $R^1$ und $R^2$ gleiche oder unterschiedliche niedere Alkylgruppen sind, wenn $R^4$ Wasserstoff bedeutet, oder in welcher $R^1$ und $R^2$ gleich oder verschieden und Wasserstoff oder niedere Alkylgruppen sind, wenn $R^4$ eine Nitrogruppe ist; und in welcher $R^3$ Wasserstoff, eine gewünschtenfalls mit einer Stickstoff enthaltenden basischen Gruppe substituierte niedere Alkylgruppe oder ein Ion eines pharmazeutisch annehmbaren Metalles ist, sowie die Säure-Additionssalze von Verbindungen der Formel (1) mit pharmazeutisch annehmbaren Säuren, falls $R^3$ eine Stickstoff enthaltende basische Gruppe aufweist.

2. Die Verbindungen des Anspruches 1, worin $R^3$ Ethyl, Propyl oder Butyl ist oder Pyridylmethyl bedeutet.

3. Die Verbindungen der Ansprüche 1 oder 2, worin $R^1$ Methyl, Ethyl oder Propyl ist.

4. Die Verbindungen von einem oder mehreren der Ansprüche 1, 2 oder 3, worin $R^2$ Methyl, Ethyl oder Propyl ist.

5. Die Verbindungen des Anspruches 1, worin $R^1$ Methyl, $R^2$ Ethyl oder Propyl, $R^3$ Ethyl oder Pyridylmethyl und $R^4$ Wasserstoff ist.

6. Die Verbindungen von einem oder mehreren der Ansprüche 2 bis 4, in welchen $R^1$ und $R^2$ unterschiedliche niedere Alkylgruppen sind, wobei diese Verbindungen als D,L-Racemat oder als D- oder L-Stereoisomere vorliegen.

7. Die Verbindungen von Anspruch 1, worin $R^1$ und $R^2$ Methylreste sind, $R^3$ eine niedere Alkylgruppe oder Pyridylmethyl und $R^4$ Wasserstoff ist.

8. Die Verbindungen von Anspruch 7, worin $R^3$ Ethyl ist.

**Patentansprüche für den Vertragsstaat: AT**

1. Verfahren zur Herstellung von Verbindungen der Formel (1)

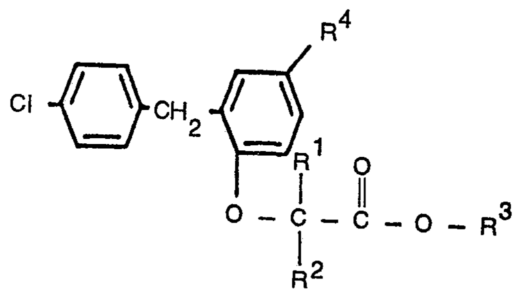

(1)

in welcher $R^1$ und $R^2$ gleich oder verschieden sind und niedere Alkylgruppen bedeuten, wenn $R^4$ Wasserstoff bedeutet, oder in welcher $R^1$ und $R^2$ gleich oder verschieden und Wasserstoff oder niedere Alkylgruppen sind, wenn $R^4$ eine Nitrogruppe ist; und in welcher $R^3$ Wasserstoff, eine gewünschtenfalls mit einer Stickstoff enthaltenden basischen Gruppe substituierte niedere Alkylgruppe oder ein Ion eines pharmazeutisch annehmbaren Metalles ist, sowie der Säure-Additionssalze von Verbindungen der Formel (1) mit pharmazeutisch annehmbaren Säuren, falls $R^3$ eine Stickstoff enthaltende basische Gruppe aufweist, dadurch gekennzeichnet, dass man ein Phenol der Formel (4)

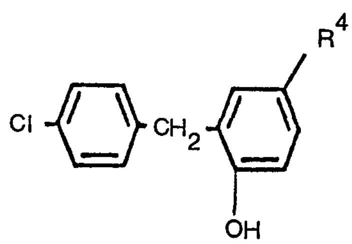

(4)

in welcher $R^4$ die oben definierte Bedeutung hat, oder ein Phenolat eines Phenols der vorstehenden Formel (4) kondensiert mit einer Verbindung der Formel (5)

$R^1$
|
Hal—C—$COOR^9$ (5)
|
$R^2$

in welcher Hal ein Halogenatom ist, $R^1$ und $R^2$ die oben definierte Bedeutung haben und $R^9$ entweder $R^3$ in der oben definierten Bedeutung oder eine in $R^3$ überführbare Gruppe ist, und dass man im letzteren Fall das Produkt anschliessend in die Zielverbindung der Formel (1) überführt.

2. Verfahren nach Anspruch 1, dadurch gekennzeichnet, dass man die Säure-Additionssalze von Verbindungen der Formel (1) mit pharmazeutisch annehmbaren Säuren herstellt, wenn $R^3$ eine Stickstoff enthaltende basische Gruppe aufweist.

3. Verfahren nach Anspruch 1, wobei $R^3$ Ethyl, Propyl oder Butyl oder Pyridylmethyl ist.

4. Verfahren nach Anspruch 1, 2 oder 3, wobei $R^1$ Methyl, Ethyl oder Propyl ist.

5. Verfahren nach einem oder mehreren der Ansprüche 1, 2, 3 oder 4, wobei $R^2$ Methyl, Ethyl oder Propyl ist.

6. Verfahren nach Anspruch 1 oder 2, wobei $R^1$ Methyl, $R^2$ Ethyl oder Propyl, $R^3$ Ethyl oder Pyridylmethyl und $R^4$ Wasserstoff ist.

7. Verfahren nach einem oder mehreren der Ansprüche 1—5, wobei $R^1$ und $R^2$ unterschiedliche niedere Alkylgruppen sind und wobei solche Verbindungen als D,L-Racemat oder in Form der D- oder L-Stereoisomere vorliegen.

8. Verfahren nach Anspruch 1 oder 2, wobei $R^1$ und $R^2$ Methylgruppen sind und $R^3$ eine niedere Alkylgruppe oder Pyridylmethyl und $R^4$ Wasserstoff ist.

9. Verfahren nach Anspruch 8, wobei $R^3$ Ethyl ist.